**Europäisches Patentamt**

**European Patent Office**

**Office européen des brevets**

(19)

(11) Veröffentlichungsnummer: **0 209 786 B1**

(12) **EUROPÄISCHE PATENTSCHRIFT**

(45) Veröffentlichungstag der Patentschrift: **10.04.91**

(51) Int. Cl.⁵: **C07K 3/20**

(21) Anmeldenummer: **86109259.1**

(22) Anmeldetag: **07.07.86**

(54) **Verfahren zur Reinigung vom humanen Tumornekrosefaktor.**

(30) Priorität: **22.07.85 DE 3526096**

(43) Veröffentlichungstag der Anmeldung:
**28.01.87 Patentblatt 87/05**

(45) Bekanntmachung des Hinweises auf die
Patenterteilung:
**10.04.91 Patentblatt 91/15**

(84) Benannte Vertragsstaaten:
**AT BE CH DE FR GB IT LI NL SE**

(56) Entgegenhaltungen:
**EP-A- 0 145 390**
**EP-A- 0 176 299**

(73) Patentinhaber: **BASF Aktiengesellschaft**
**Carl-Bosch-Strasse 38**
**W-6700 Ludwigshafen(DE)**

(72) Erfinder: **Hillen, Heinz, Dr.**
**Fasanenstrasse 10**
**W-6700 Ludwigshafen(DE)**
Erfinder: **Moesta, Peter, Dr.**
**Budapester Strasse 47**
**W-6700 Ludwigshafen(DE)**
Erfinder: **Marcinowski, Stefan, Dr.**
**Weimarer Strasse 82**
**W-6700 Ludwigshafen(DE)**

Rank Xerox (UK) Business Services

## Beschreibung

Die Erfindung betrifft ein Verfahren zur Reinigung von humanem TumorNekrose-Faktor (hTNF).

TNF wurde erstmals 1975 [Proc. Nat. Acad. Sci. USA 72, 3666 [1975]] als ein Faktor beschrieben, der Tumoren, die an Mäusen durch Induktion mit Bacillus Calmette-Guerin erzeugt werden, nekrotisiert. Die Klonierung und Expression von hTNF gelang 1984 [Nature 312 , 724 [1984]]. Nur wenig später wurde auch die Reinigung, Produktion und Charakterisierung des nativen Proteins aus einer mit Phorbolester stimulierten Makrophagenzellinie [HL60] berichtet [Biol. Chem. 260 , 2345 (1985)].

Die Expression von hTNF in rekombinanten E.coli-Stämmen führt in der Regel zu einem Gemisch aus hTNF und N-terminal Methionin-haltigem hTNF. Die Expression von hTNF in E.coli ist beispielsweise in Nature 312 , 724 (1984) beschrieben. Das nach diesem und analogen Verfahren erhältliche hTNF enthält in der Regel je nach Fermentationsbedingungen 5 bis 90 % N-terminales Methionin. Dabei ist zu beachten, daß das aktive hTNF aus drei Ketten besteht, von denen eine Kette oder mehrere Ketten an den Enden Methioninreste tragen konnen. Das durch Expression von hTNF in E.coli erhaltene hTNF enthalt also noch mono-, di- und tri-methioniniertes hTNF.

Gemäß dem nicht vorveröffentlichten EP-A 176 299 lassen sich gentechnisch hergestellte Pharmeprotene von ihren methionierten Derivaten u.a. durch Kationenaustausch-Chromatographie an einem Kationenaustauscher mit Sulfon ylgruppen trennen.

Es wurde nun ein Verfahren gefunden, mit dessen Hilfe man hTNF praktisch frei von Methionin-hTNF erhalten kann.

Gegenstand der Erfindung ist ein Verfahren zur Abtrennung von hTNF aus einem Gemisch mit N-terminal Methionin-haltigem hTNF, dadurch gekennzeichnet, daß man das Gemisch aus hTNF und N-terminal Methionin-haltigem TNF einer Kationenaustausch-Chromatographie an einem Kationenaustauscher mit $-CH_2-SO_3^{\ominus}$-Gruppen unterwirft.

Als Kationenaustauscher eignen sich insbesondere "MonoBeads, Mono S" und "S Sepharose® Fast Flow", die von der Firma Pharmacia, S-75 182 Uppsala, erhältlich sind.

Monoßeads, Mono S ist ein starker Kationenaustauscher mit $CH_2SO_3$-Gruppen. Seine Partikelgröße beträgt 9,8 $\mu$m ± 2 %, seine Ausschlußgrenze liegt bei $10^7$ Dalton. Er ist im pH-Bereich von 2 bis 12 einsetzbar. Er wird vorzugsweise in FPLC-Fertigsäulen verwendet, vgl. Prospekt "FPLC Separation Power" der Deutschen Pharmacia GmbH, D-7800 Freiburg.

"S Sepharos® Fast Flow" ist ein Austauscher auf der Basis quervernetzter Agarose (6 %), mit $-CH-SO_3$-Gegenionen, vgl. Prospekt "R and S Sepharose ® Fast Flow" der Firma Pharmacia. Er is ein starker Kationenaustauscher mit einer Kapazität von 0,18 bis 0,25 mEq/ml Gel und einer Ausschlußgrenze von $4 \times 10^6$ Dalton. Er ist im pH-Bereich von 2 bis 14 einsetzbar, vgl. Product information bulletin "R and S Sepharose® Fast Flow" der Fa. Pharmacia.

Das Gemisch aus hTNF und Methionin-haltigem hTNF (Trenngemisch) wird in einem Puffer bei pH 2,0 bis 9,5, vorzugsweise bei etwa pH 6,5 bis 8,0 auf die Saule aufgetragen. Die Elution kann mit einem pH- oder Salz-Gradienten oder einer Kombination aus beiden erfolgen. Die Säule wird vor dem Aufbringen des Gemisches mit einem Puffer äquilibriert, Nach dem Aufbringen des Gemisches wird der pH-Wert und/oder die Salzkonzentration im Elutionsmittel schrittweise, vorzugsweise aber kontinuierlich gesteigert, bis die gewunschte Fraktion die Saule verläßt.

Als Puffer können die üblicherweise in der Chromatographie eingesetzten Lösungen verwendet werden, wobei sich am besten Phosphatpuffer bewährt hat. Die Pufferkonzentration kann in weiten Grenzen (etwa 0,005 bis 0,5 molar) variieren.

Als Salze eignen sich fur die Gradienten-Elution vorzugsweise die Chloride von Alkali- und Erdalkalimetallen, sowie Ammoniumchlorid. Insbesondere eignet sich Natriumchlorid.

Das erfindungsgemäße Verfahren ermoglicht die Herstellung von hTNF in einer Reinheit von mindestens 99 %. Es erfüllt damit die Forderungen, die an Arzneimittelwirkstoffe auf der Basis von Proteinen gestellt werden.

Es ist überraschend, daß sich zwei Polypeptide, die sich nur durch einen neutralen Aminosäurerest (Methionin) unterscheiden durch Chromatographie an einem Kationenaustauscher trennen lassen. Normalerweise lassen sich so nur Substanzen voneinander trennen, die einen deutlichen Unterschied in ihrer Ladung besitzen.

Die folgenden Beispiele erläutern die Erfindung.

## Beispiel 1

Eine MonoBeads, Mono S-Säule (HR 16/10, Pharmacia) wurde mit 100 ml 20 mM Phosphatpuffer, pH 7,7, äquilibriert. 60 mg roher hTNF in 2 ml 20 mM Phosphatpuffer wurden auf die Säule gebracht. Anschließend wurde ein linearer Gradient aus 120 ml 20 mM Phosphatpuffer, pH 7,7 und 120 ml 20 mM Phosphatpuffer (pH 7,7). der 0,24 M an Natriumchlorid war, angelegt. Es wurden Peaks bei 0,10 M, 0,12 H und 0,14 H Natriumchlorid erhalten. Der Peak bei 0,14 M enthielt hTNF in einer Reinheit von 99 %. Durch Rechromatographie unter den selben Bedingungen ließ sich die Reinheit auf 99.9 % erhöhen.

Beispiel 2

Das Verfahren wurde analog Beispiel 1 durchgeführt. Statt MonoBeads, Mono S wurde S-Sepharose® Fast Flow (Pharmacia) verwendet.

Beispiel 3

Eine Säule (1,1 x 20 cm) wurde mit S Sepharose® Fast Flow gefüllt und mit 100 ml 20 mM Phosphatpuffer, pH 7,0. äquilibriert. 150 mg roher hTNF wurden in 5 ml 20 mM Phosphatpuffer, pH 7,0, gelöst und langsam (1 ml/min) auf die Säule gebracht. Danach wurde mit 30 ml 20 mM Phosphatpuffer (pH 7,0) gespült und anschließend ein linearer Gradient aus 250 ml 20 mM Phosphatpuffer, pH 7,0, und 250 ml 50 mM Phosphatpuffer, pH 9,0, angelegt. Es wurden Peaks bei pH 7,95, 8,15 und 8,5 erhalten. Der pH 8,5-Peak enthielt hTNF in einer Reinheit von 98 %. Durch Rechromatographie unter denselben Bedingungen ließ sich die Reinheit auf uber 99 % steigern.

Beispiel 4

Das Verfahren wurde analog Beispiel 3 durchgeführt. Statt S Sepharos® Fast Flow wurden jedoch Monoßeads, Mono S verwendet.

Beispiel 5

Eine Säule (1, 1x20 cm) wurde mit S Sepharose® Fast Flow (Pharmacia) gefüllt und mit 100 ml 44mM Phosphatpuffer, pH 8.34, äquilibriert. 150 mg roher hTNF wurden in 5 ml 44mM Phosphatpuffer, pH 8.34, gelöst und langsam (1 ml/min) auf die Säule gebracht. Danach wurde mit 30 ml 44mM Phosphatpuffer, pH 8,34, so lange eluiert, bis sich die UV-Absorption bei 280 nm abflachte. Dann wurde mit 50 mM Phosphatpuffer, pH 8,6, der Met-freie TNF eluiert. Er war 98 % rein. Durch Rechromatographie unter denselben Bedingungen ließ sich die Reinheit auf über 99 % steigern.

**Ansprüche**

1. Verfahren zur Abtrennung von hTNF aus einem Gemisch mit N-terminal Methionin-haltigem hTNF, dadurch gekennzeichnet, daß man das Gemisch aus hTNF und N-terminal Methionin-haltigem hTNF einer Kationenaustausch-Chromatographie an einem Kationenaustauscher mit $-CH_2-SO_3^{\ominus}$-Gruppen unterwirft.

**Claims**

1. A process for isolating hTNF from a mixture of hTNF and N-terminal methionine-containing hTNF, wherein the said mixture is subjected to cation exchange chromatography over a cation exchanger containing $-CH_2-SO_3^{\ominus}$ groups.

**Revendications**

1. Procédé de séparation de ftnh (facteur tumeurnécrose humain) d'un mélange avec ftnh contenant de la méthionine à N terminal, caractérisé par le fait que l'on soumet le mélange de ftnh et de ftnh contenant de la méthionine à N terminal à une chromatographie d'échange de cations au contact d'un échangeur de cations à groupes $-CH_2-SO_3^{\ominus}$.